(19) Europäisches Patentamt
European Patent Office
Office européen des brevets

(11) EP 2 147 941 A1

(12) DEMANDE DE BREVET EUROPEEN

(43) Date de publication:
**27.01.2010 Bulletin 2010/04**

(21) Numéro de dépôt: **09163112.7**

(22) Date de dépôt: **18.06.2009**

(51) Int Cl.:
***C08F 290/06*** *(2006.01)* ***C08F 293/00*** *(2006.01)*
***C08L 53/00*** *(2006.01)* ***A61K 8/90*** *(2006.01)*
***A61Q 1/00*** *(2006.01)* ***A61Q 3/02*** *(2006.01)*
***A61Q 5/00*** *(2006.01)*

(84) Etats contractants désignés:
**AT BE BG CH CY CZ DE DK EE ES FI FR GB GR HR HU IE IS IT LI LT LU LV MC MK MT NL NO PL PT RO SE SI SK TR**
Etats d'extension désignés:
**AL BA RS**

(30) Priorité: **24.07.2008 FR 0855070**

(71) Demandeur: **L'Oréal**
**75008 Paris (FR)**

(72) Inventeur: **Farcet, Céline**
**75012, PARIS (FR)**

(74) Mandataire: **Dodin, Catherine et al**
**L'Oréal**
**D.I.P.I.**
**25-29 Quai Aulagnier**
**92600 Asnières (FR)**

# (54) Polymère séquencé, composition cosmétique le comprenant et procédé de traitement cosmétique

(57) L'invention concerne un polymère séquencé comprenant, dans une séquence de Tg supérieure à 20°C, au moins un monomère de formule (I):

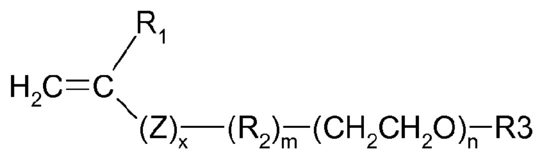

(I)

dans laquelle :
- Z est de préférence COO ou CONH,
- R2 est un radical divalent en C1-C30,
- n est un entier compris entre 3 et 300 inclus;
- R3 est un atome d'hydrogène ou un radical carboné en C1-C30.

L'invention concerne également une composition cosmétique comprenant, dans un milieu cosmétiquement acceptable, ledit polymère séquencé, ainsi qu'un procédé de traitement cosmétique des matières kératiniques employant ladite composition, et tout particulièrement un procédé de maquillage des lèvres.

EP 2 147 941 A1

## Description

Divers types de polymères sont conventionnellement utilisés dans les compositions cosmétiques en raison des diverses propriétés qu'ils peuvent leur apporter. Ils sont par exemple utilisés dans des compositions de maquillage ou de soin de la peau, des lèvres ou des phanères, telles que des vernis à ongles ou des compositions pour les cheveux.
Cependant, en utilisant au sein d'une même composition deux polymères incompatibles, c'est à dire non miscibles dans un même solvant, le formulateur est confronté, du fait de l'incompatibilité des polymères, à des problèmes de séparation de phases, voire de décantation, et de manière générale à l'obtention d'une composition non homogène. Ces problèmes ne pouvaient être jusqu'ici résolus que par la présence dans la composition d'un composé permettant de compatibiliser les polymères entre eux.
Pour pallier ce problème, il a été proposé, dans la demande EP1411069, des polymères de structure particulière, comprenant au moins deux séquences incompatibles entre elles et reliées par une séquence intermédiaire qui comprend au moins un monomère constitutif de chacune des deux séquences.
Cette demande décrit principalement de polymères séquencés préparés à partir de monomères de type acrylates et méthacrylates d'alkyle, notamment de méthyle, d'isobutyle, d'isobornyle, de trifluoroéthyle, ou d'acide (méth)acrylique. Ces polymères sont généralement véhiculables, notamment solubles, dans des solvants organiques tels que les esters courts (acétate de butyle ou d'éthyle), les alcools courts tels que l'éthanol, ou les alcanes aliphatiques tels que l'isododécane. Ils permettent d'obtenir des films brillants et peu collants, particulièrement appréciés dans certains domaines cosmétiques.
Toutefois, ces films présentent un certain caractère cassant ainsi qu'une sensibilité aux corps gras, notamment au cours du temps.
Or, on recherche, notamment dans le domaine du maquillage, des polymères susceptibles de présenter une résistance et une tenue aux agressions externes, notamment aux 'agressions' par des corps gras, tels que par exemple par l'huile alimentaire ou le sébum.

De plus, ces polymères ne permettent pas d'obtenir des propriétés cosmétiques optimales, notamment en terme de glissant, de non collant, de confort, de tenue de la composition et de toucher.

La demanderesse a découvert de manière surprenante de nouveaux polymères, véhiculables en milieu organique, permettant d'obtenir de bonnes propriétés cosmétiques telles qu'une bonne adhésion sur le support (peau ou cheveu), donc une bonne tenue de la composition cosmétique.

Un objet de la présente invention est un polymère séquencé comprenant au moins une première séquence et au moins une deuxième séquence, incompatibles l'une avec l'autre, dans lequel l'une des séquences présente une température de transition vitreuse (Tg) strictement supérieure à 20°C et comprend 0,5 à 13% en poids, par rapport au poids de ladite séquence, d'au moins un monomère de formule (I) telle que défini ci-après.

L'invention a également pour objet la composition cosmétique comprenant, dans un milieu cosmétiquement acceptable, ledit polymère.

Le confort de la composition cosmétique est amélioré, ainsi que son toucher, sa douceur et son glissant, et son caractère non collant.
La composition permet d'obtenir un film brillant et peu collant, tout en étant en outre peu ou non cassant et suffisamment souple, et présentant avantageusement une bonne résistance aux corps gras.

On peut penser que la présence de motifs PEG dans le polymère permet d'apporter à la fois du glissant, de l'insensibilité aux huiles, c'est-à-dire une amélioration du confort et de la tenue, ainsi que du volume et du brillant.
Les polymères à motifs PEG présentent des propriétés de confort accrues grâce au glissant et à la lubrification des motifs PEG qui se développe en environnement humide.
Les compositions selon l'invention peuvent apporter, dans le domaine du maquillage, des propriétés de confort accrues, notamment un glissant amélioré, en particulier en environnement humide. De plus, elles peuvent présenter une résistance améliorée aux agressions externes (repas, sébum) et aux frottements. Le confort et la tenue sont donc améliorés.
Dans le cas plus particulier des rouges à lèvres, l'utilisation de la salive sur les lèvres peut permettre de 'relubrifier' pour prolonger le confort et la tenue, et également de générer un gonflement, ou du volume, ce qui donnera un effet repulpeur; le film humide ainsi créé en surface protège le dépôt; les polymères selon l'invention peuvent donc apporter du volume en gonflant en présence d'eau, ainsi qu'une brillance 'mouillée'.

De plus, les polymères sont véhiculables dans des milieux huileux particulièrement prisés dans le domaine du maquillage; or, les polymères comprenant des motifs PEG (polyéthylèneglycol) étaient jusqu'à présent généralement employés en solution ou dispersion aqueuse, milieu plus difficilement formulable dans le domaine du maquillage.

La composition selon l'invention comprend donc au moins un polymère séquencé comprenant au moins une première séquence et au moins une deuxième séquence, qui peuvent avoir des températures de transition vitreuse (Tg) différentes et qui sont avantageusement incompatibles l'une avec l'autre.
On précise que les termes "première" et "deuxième" séquences ne conditionnent nullement l'ordre desdites séquences (ou blocs) dans la structure du polymère.

Par "séquences incompatibles l'une avec l'autre", on entend que le mélange formé du polymère correspondant à la première séquence et du polymère correspondant à la deuxième séquence, n'est pas miscible dans le solvant de polymérisation majoritaire en poids du polymère séquencé, à température ambiante (25°C) et pression atmosphérique ($10^5$ Pa), pour une teneur du mélange de polymères supérieure ou égale à 5% en poids, par rapport au poids total du mélange (polymères et solvant), étant entendu que :

i) lesdits polymères sont présents dans le mélange en une teneur telle que le rapport pondéral respectif va de 10/90 à 90/10, et que
ii) chacun des polymères correspondant à la première et seconde séquences a une masse moléculaire moyenne (en poids ou en nombre) égale à celle du polymère séquencé +/- 15%.
Dans le cas d'un mélange de solvants de polymérisation, dans l'hypothèse de deux ou plusieurs solvants présents, ledit mélange de polymères est non miscible dans au moins l'un d'entre eux. Bien entendu, dans le cas d'une polymérisation réalisée dans un solvant unique, ce dernier est le solvant majoritaire.

Lesdites première et deuxième séquences peuvent être avantageusement reliées entre elles par un segment intermédiaire comprenant au moins un monomère m1 constitutif de la première séquence et au moins un monomère m2 constitutif de la deuxième séquence. Préférentiellement, ledit segment intermédiaire forme une séquence (ou bloc) intermédiaire. De préférence, m2 est différent de m1. Ledit segment ou séquence intermédiaire peut notamment permettre de "compatibiliser" ces première et deuxième séquences.
Le polymère séquencé employé dans le cadre de l'invention est avantageusement un polymère éthylénique séquencé, linéaire, ramifié ou greffé, de préférence linéaire, et susceptible de former un dépôt, plus particulièrement un film (filmogène).
Par polymère éthylénique, on entend un polymère obtenu par polymérisation de monomères comprenant une insaturation éthylénique.
Par polymère séquencé, on entend un polymère comprenant au moins 2 séquences distinctes, de préférence au moins 3 séquences distinctes; de préférence diblocs ou triblocs.
Le polymère selon l'invention est linéaire, ramifié ou greffé; de préférence il ne comprend pas de monomère ou composé multifonctionnel, ajouté volontairement, et susceptible de générer des embranchements et/ou des réticulations.
Par polymère formant un dépôt, on entend un polymère apte à former à lui seul ou en présence d'un agent auxiliaire, un dépôt adhérent sur un support, notamment sur les matières kératiniques.
Par polymère filmogène, on entend un polymère apte à former à lui seul ou en présence d'un agent auxiliaire de filmification, un film continu et adhérent sur un support, notamment sur les matières kératiniques.
Chaque séquence, ou bloc, du polymère selon l'invention est issue d'un type de monomère ou de plusieurs types de monomères différents. Cela signifie que chaque séquence peut être constituée d'un homopolymère ou d'un copolymère, qui peut être statistique, alterné ou autre.
Avantageusement, lorsqu'il est présent, le segment ou la séquence intermédiaire comprenant au moins un monomère constitutif de la première séquence et au moins un monomère constitutif de la deuxième séquence du polymère est un polymère statistique. De préférence, ledit segment ou ladite séquence intermédiaire est issu essentiellement de monomères constitutifs de la première séquence et de la deuxième séquence. Par "essentiellement", on entend au moins à 85%, de préférence au moins à 90%, mieux à 95% et encore mieux à 100%.
Selon l'invention, les première et deuxième séquences ont de préférence des températures de transition vitreuse différentes, avec un écart généralement supérieur à 5°C, de préférence supérieur à 10°C, et mieux supérieur à 20°C. Avantageusement, la séquence intermédiaire a une température de transition vitreuse Tg comprise entre les températures de transition vitreuse des première et deuxième séquences.
Les températures de transition vitreuse indiquées sont, sauf indication contraire, des Tg théoriques déterminées à partir des Tg théoriques des monomères constitutifs de chacune des séquences, que l'on peut trouver dans un manuel de référence tel que le Polymer Handbook, 4th éd. (Brandrup, Immergut, Grulke), 1999, John Wiley, selon la relation suivante, dite Loi de Fox :

$$\frac{1}{Tg} = \sum_i \left(\frac{\varpi i}{Tgi}\right)$$

wi étant la fraction massique du monomère i dans la séquence considérée et Tgi étant la température de transition vitreuse de l'homopolymère du monomère i (exprimée en Kelvin).
Le polymère séquencé selon l'invention comprend donc au moins une séquence ayant une Tg inférieure ou égale à 20°C, et de préférence au moins une séquence ayant une Tg strictement supérieure à 20°C.
De préférence, l'une des séquences a une Tg comprise entre 20°C (exclu) et 160°C, notamment comprise

entre 40°C et 120°C, préférentiellement comprise entre 50°C et 110°C.
De préférence, l'une des séquences a une Tg comprise entre -150°C et 20°C (in-clu), notamment comprise entre -100°C et 10°C, préférentiellement comprise entre -50°C et 0°C.

De préférence, la séquence ayant la Tg la plus élevée est en quantité majoritaire, en poids, par rapport aux autres séquences.

De préférence, le polymère séquencé selon l'invention est constitué exclusivement d'une première et d'une deuxième séquence (c'est alors un dibloc), ou bien exclusivement d'une première séquence, d'une deuxième séquence et d'un segment intermédiaire qui est de préférence une séquence intermédiaire (ce peut être donc un tribloc).

Lorsque le polymère séquencé ne comprend pas de séquence ou segment intermédiaire (dibloc), la séquence ayant la Tg la plus élevée peut être présente dans le polymère séquencé en une quantité de 50 à 90% en poids, notamment 55 à 80% et encore mieux de 60 à 75% en poids, par rapport au poids total du polymère séquencé et la deuxième séquence peut être présente en une quantité de 10 à 50% en poids, notamment 20 à 45% en poids et encore mieux de 25 à 40% en poids, par rapport au poids total du polymère séquencé.

Lorsque le polymère séquencé comprend une séquence ou segment intermédiaire, la séquence ayant la Tg la plus élevée peut être présente dans le polymère séquencé en une quantité de 45 à 90% en poids, notamment 50 à 80% et encore mieux de 55 à 75% en poids, par rapport au poids total du polymère séquencé; la deuxième séquence peut être présente en une quantité de 9 à 45% en poids, notamment 10 à 40% en poids et encore mieux de 20 à 35% en poids, par rapport au poids total du polymère séquencé; le segment, ou séquence, intermédiaire peut représenter 1 à 10% en poids, notamment 2 à 7% en poids et encore mieux 3 à 5% en poids, du poids total du polymère séquencé.

Lesdites première et/ou deuxième séquences peuvent être constituées d'un homopolymère ou d'un copolymère, qui peut être statistique, alterné ou autre, de préférence statistique.
La nature chimique et/ou la quantité des monomères constituants chacune des séquences peuvent bien évidement être choisies par l'homme du métier, sur la base de ses connaissances générales, pour obtenir des séquences ayant les Tg requises.

La caractéristique essentielle des polymères selon l'invention est que la séquence de Tg strictement supérieure à 20°C comprend 0,5 à 13% en poids, par rapport au poids de cette séquence, d'au moins un monomère de formule (I), ou un mélange de tels monomères :

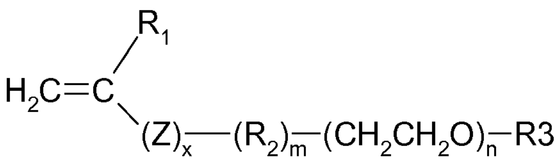

(I)

dans laquelle :

- R1 est un atome d'hydrogène ou un radical méthyle;
- Z est un groupement divalent choisi parmi -COO-, -CONH-, $-CONCH_3-$, -OCO-, - O-, $-SO_2-$ -CO-O-CO- ou $-CO-CH_2-CO-$;
- x est 0 ou 1;
- R2 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;
- m est 0 ou 1;
- n est un entier compris entre 3 et 300 inclus;
- R3 est un atome d'hydrogène ou un radical carboné, saturé ou insaturé, éventuellement aromatique, linéaire, ramifié ou cyclique, de 1 à 30 atomes de carbone, pouvant comprendre 1 à 20 hétéroatomes choisis parmi O, N, S, F, Si et P.

De préférence, x = 1 et Z représente COO ou CONH, préférentiellement COO.

Dans le radical R2, le ou les hétéroatomes, quand ils sont présents, peuvent être intercalés dans la chaîne dudit radical R2, ou bien ledit radical R2 peut être substitué par un ou plusieurs groupes les comprenant tels que hydroxy, amino (NH2, NHR' ou NR'R" avec R' et R" identiques ou différents représentant un alkyle linéaire ou ramifié en C1-C22, notamment méthyle ou éthyle), -CF3, -CN, -SO3H ou -COOH.
En particulier, R2 peut comprendre un groupement -O-, -N(R)-, -CO- et leur combinaison, et notamment -O-CO-O-, -CO-O-, -N(R)CO-; -O-CO-NR-, -NR-CO-NR-, avec R représentant H ou un alkyle linéaire ou ramifié en C1-C22, comprenant éventuellement 1 à 12 hétéroatomes choisis parmi O, N, S, F, Cl, Br, Si et P.

Notamment R2 peut être :

- un radical alkylène ayant 1 à 20 atomes de carbone, tel que méthylène, éthylène, n-propylène, isopropylène, n-butylène, isobutylène, tertiobutylène, pentylène, isopentylène, n-hexylène, isohexylène, heptylène, isoheptylène, n-octylène, isooctylène, nonylène, isononylène, décylène, isodécylène, n-dodécylène, isododécylène, tridécylène, n-tétradécylène, hexadécylène, n-octadécylène, docosanylène, arachinylène;
- un radical cycloalkylène ayant 5 à 10 atomes de carbone, substitué ou non, tel que cyclopentylène, cyclohexylène, cycloheptylène, cyclooctylne, cyclononylène, cyclodécylène;
- un radical phénylène $-C_6H_4-$(ortho, méta ou para) éventuellement substitué, par un radical alkyle en C1-C12 comprenant éventuellement 1 à 18 hétéroatomes choisis parmi O, N, S, F, Si et P;
- un radical benzylène $-C_6H_4-CH_2-$ éventuellement substitué, par un radical alkyle en C1-C12 comprenant éventuellement 1 à 18 hétéroatomes choisis parmi O, N, S, F, Si et P;
- un radical de formule $-CH_2-O-CO-O-$, $CH_2-CH_2-O-CO-O-$, $-CH_2-CO-O-$, $-CH_2-CH_2-CO-O-$, $-CH_2-O-CO-NH-$, $-CH_2-CH_2-O-CO-NH-$; $-CH_2-NH-CO-NH-$, $-CH_2-CH_2-NH-CO-NH-$ ;$-CH_2-CHOH-$, $-CH_2-CH_2-CHOH-$, $-CH_2-CH_2-CH(NH_2)-$, $-CH_2-CH(NH_2)-$, $-CH_2-CH_2-CH(NHR')-$, $-CH_2-CH(NHR')-$, $-CH_2-CH_2-CH(NR'R'')-$, $-CH_2-CH(NR'R'')-$, $-CH_2-CH_2-CH_2-NR'-$, $-CH_2-CH_2-CH_2-O-$; $-CH_2-CH_2-CHR'-O-$ avec R' et R'' représentant un alkyle linéaire ou ramifié en C1-C22 comprenant éventuellement 1 à 12 hétéroatomes choisis parmi O, N, S, F, Si et P;
- ou un mélange de ces radicaux.

De préférence R2 peut être :

- un radical alkylène ayant 1 à 20 atomes de carbone, notamment méthylène, éthylène, n-propylène, n-butylène, n-hexylène, n-octylène, n-dodécylène, n-octadécylène;
- un radical phénylène $-C_6H_4-$(ortho, méta ou para) éventuellement substitué, par un radical alkyle en C1-C12 comprenant éventuellement 1 à 18 hétéroatomes choisis parmi O, N, S, F, Si et P; ou
- un radical benzylène $-C_6H_4-CH_2-$ éventuellement substitué par un radical alkyle en C1-C12 comprenant éventuellement 1 à 18 hétéroatomes choisis parmi O, N, S, F, Si et P.

De préférence, n est compris entre 5 et 200 inclus, et encore mieux entre 6 et 120 inclus, voire entre 7 et 50 inclus.

De préférence, R3 est un atome d'hydrogène; un radical phényle éventuellement substitué par un radical alkyle en C1-C12 comprenant éventuellement 1 à 20 hétéroatomes choisis parmi O, N, S, F, Si et P; un radical alkyle en C1-C30, notamment C1-C22, voire C2-C16, comprenant éventuellement 1 à 18 hétéroatomes choisis parmi O, N, S, F, Si et P; un radical cycloalkyle en C3-C12, notamment C4-C8, voire C5-C6, comprenant éventuellement 1 à 18 hétéroatomes choisis parmi O, N, S, F, Si et P.

Parmi les radicaux R3, on peut citer les chaînes méthyle, éthyle, propyle, benzyle, éthylhexyle, lauryle, stéaryle, béhényle ($-(CH_2)_{21}-CH_3$), et également les chaînes alkyles fluorées telles que par exemple heptadecafluorooctyl sulfonyl amino éthyle $CF_3-(CF_2)_7-SO_2-N(C_2H_5)-CH_2-CH_2$, ou encore les chaînes $-CH_2-CH_2-CN$, succini-mido, maléimido, mésityle, tosyle, triéthoxysilane ou phtalimide.

Préférentiellement, les monomères de formule (I) sont tels que :

- x=1 et Z représente COO,
- m=0,
- n= 6 à 120 inclus,
- R3 est choisi parmi un atome d'hydrogène; un radical phényle éventuellement substitué par un radical alkyle en C1-C12; un radical alkyle en C1-C30, notamment C1-C22, voire C2-C16.

De préférence, les monomères de formule (I) ont un poids moléculaire compris entre 300 et 5000 g/mol.

Parmi les monomères de formule (I) particulièrement préférés, on peut citer :

- le (méth)acrylate de poly(éthylène glycol) dans lequel R1 est H ou méthyle; Z est COO, x = 1, m=0 et R3 = H;
- le (méth)acrylate de méthyl-poly(éthylène glycol), aussi appelé (méth)acrylate de méthoxy-poly(éthylèneglycol), dans lequel R1 est H ou méthyle, Z est COO, x = 1, m=0 et R3 = méthyle;
- les (méth)acrylate d'alkyl-poly(éthylène glycol) dans lequel R1 est H ou méthyle, Z est COO, x = 1, m=0 et R3 = alkyl.
- les (méth)acrylates de phényl-poly(éthylène glycol), aussi appelé (méth)acrylate de poly(éthylène glycol) phényl éther, dans lequel R1 est H ou méthyl, Z est COO, x = 1, m=0 et R3 = phényle.

Des exemples de monomères commerciaux sont :

- le CD 350 (méthacrylate de méthoxy-poly(éthylène glycol 350) et le CD 550 (méthacrylate de méthoxy-poly(éthylène glycol 550), fourni par SARTOMER Chemicals;
- le M90G (méthacrylate de méthoxy-poly(éthylène glycol (9 unités de répétition)) et le M230G (méthacrylate de méthoxy-polyéthylène glycol (23 unités de répétitions)) disponibles chez Shin-Nakamura Chemicals;
- les méthacrylates de méthoxy-poly(éthylène glycol) de poids moléculaires moyens 300, 475 ou 1100, disponibles chez Sigma-Aldrich;
- l'acrylate de méthoxy-poly(éthylène glycol) de poids moléculaire moyen 426 disponible chez Sigma-Aldrich;
- les méthacrylates de méthoxy-poly(éthylène glycol) disponibles chez LAPORTE sous les dénominations commerciales : MPEG 350, MPEG 550, S10W, S20W, ou chez Cognis sous la dénomination BISOMER.
- les poly(éthylène glycol) monomethyl éther, mono(succinimidyl succinate) ester de poids moléculaire moyen 1900 ou 5000, de chez Polysciences;
- le méthacrylate de béhényl poly(éthylèneglycol PEG-25), disponible chez Rhodia, sous la dénomination SIPOMER BEM;
- les acrylates de poly(éthylène glycol) phényl éther de poids moléculaires moyens 236, 280 ou 324 disponibles chez Aldrich;
- le méthoxy polyéthylène glycol 5000 2-(vinyl sulfonyl) éthyl éther disponible commercialement chez Fluka;
- le méthacrylate de polyéthylène glycol éthyl éther disponible chez Aldrich ;
- les méthacrylates de polyéthylène glycol 8000, 4000, 2000 de Monomer & Polymer Dajac laboratories.
- le methacrylate de méthoxy-poly(éthylène glycol) 2000 Norsocryl 402 d'Arkema
- le methacrylate de méthoxy-poly(éthylène glycol) 5000 Norsocryl 405 d'Arkema
- le poly(éthylène glycol) méthyléther acrylate d'Aldrich, Mn=454 g/mol, DP = 8-9

Les monomères de formule (I) tout particulièrement préférés sont choisis parmi les (méth)acrylates de poly (éthylène glycol) et les (méth)acrylates d'alkylpoly(éthylène glycol), plus particulièrement les méthacrylates de méthylpoly (éthylène glycol).

De préférence, le monomère de formule (I) ou le mélange de tels monomères, représente 1 à 12% en poids, notamment 2 à 11 % en poids, voire 3 à 10% en poids, du poids total de monomères servant à former la séquence de Tg strictement supérieure à 20°C.

Le monomère de formule (I), seul ou en mélange, est présent uniquement dans la séquence de Tg strictement supérieure à 20°C; il n'est pas présent dans une autre séquence, notamment dans une séquence de Tg inférieure ou égale à 20°C.

Les autres monomères susceptibles d'être présents dans le polymère, donc constituants partie de la séquence de Tg supérieure à 20°C et tout ou partie des autres séquences, peuvent être choisis parmi, seuls ou en mélange, les monomères suivants :

- (i) les hydrocarbures éthyléniques ayant 2 à 10 carbones, tels que l'éthylène, l'isoprène, ou le butadiène;

- (ii) les (méth)acrylates de formule $CH_2=CHCOOR_3$ ou $CH_2=C(CH_3)COOR_3$ dans lesquelles $R_3$ représente :

    - un groupe alkyle linéaire ou ramifié, saturé ou insaturé, ayant 1 à 22 atomes de carbone, notamment 4 à 20, voire 6 à 18 atomes de carbone, pouvant éventuellement comprendre, intercalé et/ou en substitution, un ou plusieurs hétéroatomes choisis parmi O, N, S, P et les atomes d'halogène (Cl, Br, I et F);
    notamment R3 peut être un groupe méthyle, éthyle, propyle, n-butyle, isobutyle, tertiobutyle, hexyle, éthylhexyle, octyle, lauryle, isooctyle, isodécyle, dodécyle, béhényle, stéaryle; éthyl-2-perfluorohexyle; ou un groupe hydroxyalkyle en $C_{1-4}$ tel que 2-hydroxyéthyle, 2-hydroxybutyle et 2-hydroxypropyle; ou un groupe alcoxy ($C_{1-4}$) alkyle ($C_{1-4}$) tel que méthoxyéthyle, éthoxyéthyle et méthoxypropyle,
    - un groupe cycloalkyle en $C_3$ à $C_{12}$, tel que le groupe isobornyle, cyclohexyle, t-butylcyclohexyle,
    - un groupe aryle en $C_3$ à $C_{20}$ tel que le groupe phényle,
    - un groupe aralkyle en $C_4$ à $C_{30}$ (groupe alkyle en $C_1$ à $C_8$) tel que 2-phényl-éthyle, t-butylbenzyle ou benzyle,
    - un groupe hétérocyclique de 4 à 12 chaînons contenant un ou plusieurs hétéroatomes choisis parmi O, N, et S, le cycle étant aromatique ou non,
    - un groupe hétérocycloalkyle (alkyle de 1 à 4 C), tel que furfurylméthyle ou tétrahydrofurfurylméthyle,
    lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles en C1-C4, linéaires ou ramifiés, qui peuvent éventuellement comprendre, intercalé et/ou en substitution, un ou plusieurs hétéroatomes choisis parmi O, N, S, P et les atomes d'halogène (Cl, Br, I et F);

- (iii) les (méth)acrylamides de formule $CH_2=CHCONR_6R_7$ ou $CH_2=C(CH_3)CON\ R_6R_7$ dans laquelle $R_6$ et $R_7$, iden-

tiques ou différents, représentent :

- un atome d'hydrogène; ou
- un groupe alkyle linéaire ou ramifié, saturé ou insaturé, ayant 1 à 22 atomes de carbone, notamment 4 à 20, voire 6 à 18 atomes de carbone, pouvant éventuellement comprendre, intercalé et/ou en substitution, un ou plusieurs hétéroatomes choisis parmi O, N, S, P et les atomes d'halogène (Cl, Br, I et F);
  notamment R6 et/ou R7 peut être un groupe méthyle, éthyle, propyle, n-butyle, isobutyle, tertiobutyle, hexyle, éthylhexyle, octyle, lauryle, isooctyle, isodécyle, dodécyle, béhényle, stéaryle; éthyl-2-perfluorohexyle; ou un groupe hydroxyalkyle en $C_{1-4}$ tel que 2-hydroxyéthyle, 2-hydroxybutyle et 2-hydroxypropyle; ou un groupe alcoxy($C_{1-4}$) alkyle($C_{1-4}$) tel que méthoxyéthyle, éthoxyéthyle et méthoxypropyle,
- un groupe cycloalkyle en $C_3$ à $C_{12}$, tel que le groupe isobornyle, cyclohexyle, t-butylcyclohexyle;
- un groupe aryle en $C_3$ à $C_{20}$ tel que le groupe phényle,
- un groupe aralkyle en $C_4$ à $C_{30}$ (groupe alkyle en $C_1$ à $C_8$) tel que 2-phényl-éthyle, t-butylbenzyle ou benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle en C1-C4), tel que furfurylméthyle ou tétra-hydrofurfurylméthyle,
  lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles en C1-C4, linéaires ou ramifiés, qui peuvent éventuellement comprendre, intercalé et/ou en substitution, un ou plusieurs hétéroatomes choisis parmi O, N, S, P et les atomes d'halogène (Cl, Br, I et F);
  On peut notamment citer le (méth)acrylamide, le N-éthyl(méth)acrylamide, le N-butyl(méth)acrylamide, le N-t-butyl(méth)acrylamide, le N-isopropyl(méth)acrylamide, le N,N-diméthyl(méth)acrylamide, le N,N-dibutyl(méth)acrylamide, le N-octyl(méth)acrylamide, le N-dodécyl(méth)acrylamide, le N-undécyl(méth)acrylamide et le N-2-hydroxypropyl(méth)acrylamide.

- (iv) les composés vinyliques de formule $CH_2$=CH-$R_9$, $CH_2$=CH-$CH_2$-$R_9$ ou $CH_2$=C($CH_3$)-$CH_2$-$R_9$

dans lesquelles $R_9$ est un groupe hydroxyle, halogène (Cl ou F), $NH_2$, $OR_{14}$ où $R_{14}$ représente un groupe phényle ou un groupe alkyle en $C_1$ à $C_{12}$ (le monomère est un éther de vinyle ou d'allyle); acétamide ($NHCOCH_3$); un groupe $OCOR_{15}$ où $R_{15}$ représente un groupe alkyle de 2 à 12 carbones, linéaire ou ramifié (le monomère est un ester vinylique ou d'allyle); ou un groupe choisi parmi :

- un groupe alkyle linéaire ou ramifié, de 1 à 22 atomes de carbone, notamment 4 à 20, voire 6 à 18 atomes de carbone, pouvant éventuellement comprendre, intercalé et/ou en substitution, un ou plusieurs hétéroatomes choisis parmi O, N, S, P et les atomes d'halogène (Cl, Br, I et F);
- un groupe cycloalkyle en $C_3$ à $C_{12}$ tel que isobornyle, cyclohexyle,
- un groupe aryle en $C_3$ à $C_{20}$ tel que phényle,
- un groupe aralkyle en $C_4$ à $C_{30}$ (groupe alkyle en $C_1$ à $C_8$) tel que 2-phényléthyle ; benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle en C1-C4), tel que furfurylméthyle ou tétrahydrofurfurylméthyle,
  lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène et les groupes alkyles en C1-C4, linéaires ou ramifiés, qui peuvent éventuellement comprendre, intercalé et/ou en substitution, un ou plusieurs hétéroatomes choisis parmi O, N, S, P et les atomes d'halogène (Cl, Br, I et F);
  On peut citer le vinylcyclohexane, le styrène; l'acétate de vinyle, le propionate de vinyle, le butyrate de vinyle, l'éthylhexanoate de vinyle, le néononanoate de vinyle et le néododécanoate de vinyle; le vinylméthyléther, le vinyléthyléther et le vinylisobutyléther.
- (v) les monomères à insaturation(s) éthylénique(s) comprenant au moins une fonction acide carboxylique, phosphorique ou sulfonique, ou anhydride, comme par exemple l'acide acrylique, l'acide méthacrylique, l'acide crotonique, l'anhydride maléique, l'acide itaconique, l'acide fumarique, l'acide maléique, l'acide acrylami-dopropanesulfonique, l'acide vinylbenzoïque, l'acide vinylphosphorique et les sels de ceux-ci,
- (vi) les monomères à insaturation(s) éthylénique(s) comprenant au moins une fonction amine tertiaire comme la 2-vinylpyridine, la 4-vinylpyridine, le méthacrylate de diméthylaminoéthyle, le (méth)acrylate de diéthylaminoéthyle, le diméthy-laminopropyl(méth)acrylamide et les sels de ceux-ci.

Les sels peuvent être formés par neutralisation des groupes anioniques à l'aide d'une base minérale, telle que LiOH, NaOH, KOH, $Ca(OH)_2$, $NH_4OH$ ou $Zn(OH)_2$; ou par une base organique telle qu'une alkylamine primaire, secondaire ou tertiaire, notamment la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine et la diméthylamino-2-propanol. On peut encore citer la lysine ou la 3-(diméthylamino)propylamine.

On peut également peut citer les sels d'acides minéraux, tels que l'acide sulfurique, l'acide chlorhydrique, l'acide bromhydrique, l'acide iodhydrique, l'acide phosphorique, l'acide borique. On peut aussi citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Parmi les monomères dont les homopolymères ont une Tg supérieure à 20°C, susceptibles notamment d'être présents dans la séquence de Tg supérieure à 20°C, on peut notamment citer :

- les méthacrylates de formule : $CH_2=C(CH_3)-COOR_1$

dans laquelle $R_1$ représente un groupe alkyle non substitué, linéaire ou ramifié, contenant de 1 à 4 atomes de carbone, tel qu'un groupe méthyle, éthyle, propyle ou isobutyle, ou bien $R_1$ représente un groupe cycloalkyle $C_4$ à $C_{12}$, notamment isobornyle;

- les acrylates de formule : $CH_2=CH-COOR_2$

dans laquelle $R_2$ représente un groupe tertiobutyle ou un groupe cycloalkyle en $C_4$ à $C_{12}$ tel que un groupe isobornyle;

- les (méth)acrylamides de formule : $CH_2 = CR'-CO-NR^7R^8$
  avec R' représentant H ou $CH_3$, et $R_7$ et $R_8$, identiques ou différents, représentent un atome d'hydrogène ou un groupe alkyle en $C_1$ à $C_{12}$ linéaire ou ramifié, tel qu'un groupe n-butyle, t-butyle, isopropyle, isohexyle, isooctyle, ou isononyle; ou encore $R_7$ représente H et $R_8$ représente un groupement 1,1-diméthyl-3-oxobutyle,
- l'acide méthacrylique et l'acide acrylique.
  On peut tout particulièrement citer le méthacrylate de méthyle, le méthacrylate d'éthyle, le méthacrylate d'isobutyle, le (méth)acrylate de tertio-butyle, l'acide (méth)acrylique, le (méth)acrylate d'isobornyle, le N-butylacrylamide, le N-t-butylacrylamide, le N-isopropylacrylamide, le N,N-diméthylacrylamide, le N,N-dibutylacrylamide, le méthacrylate de n-butyle, l'acrylate de cyclodécyle, l'acrylate de néopentyle, l'isodécylacrylamide, et leurs mélanges.

Parmi les monomères dont les homopolymères ont une Tg inférieure à 20°C, susceptibles notamment d'être présents dans la séquence de Tg supérieure à 20°C, on peut notamment citer :

- les acrylates de formule $CH_2=CHCOOR_3$, dans laquelle $R_3$ représente un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S,
- les méthacrylates de formule $CH_2=C(CH_3)-COOR_4$, dans laquelle $R_4$ représente un groupe alkyle non substitué en $C_6$ à $C_{12}$ linéaire ou ramifié, dans lequel se trouve(nt) éventuellement intercalé(s) un ou plusieurs hétéroatomes choisis parmi O, N et S;
- les esters vinyliques de formule $R_5-CO-O-CH=CH_2$ où $R_5$ représente un groupe alkyle en $C_4$ à $C_{12}$ linéaire ou ramifié ;
- les ($C_4$-$C_{12}$alkyl)-vinyléthers, notamment le méthylvinyléther et l'éthylvinyléther;
- les N-($C_4$-$C_{12}$ alkyl)acrylamides, tels que le N-octylacrylamide,
- et leurs mélanges.
  On peut tout particulièrement citer l'acrylate de méthyle, l'acrylate d'éthyle, l'acrylate d'isobutyle, le (méth)acrylate d'éthyl-2-hexyle et leurs mélanges.

Dans un mode de réalisation préféré, le polymère selon l'invention comprend dans au moins une séquence, préférentiellement dans chacune des séquences, au moins un monomère choisi parmi les esters d'acide (méth)acrylique; éventuellement, il peut comprendre en outre au moins un deuxième monomère choisi parmi l'acide acrylique et l'acide méthacrylique, voire leur mélange.

Préférentiellement, tous les monomères constituants le polymère séquencé sont choisis parmi les esters d'acide (méth) acrylique et l'acide (méth)acrylique.

Ainsi, on choisit plus particulièrement les monomères parmi, seuls ou en mélange :

- les (méth)acrylates d'alkyle en C1-C22, notamment C4-C20, voire C6-C18, ou de cycloalkyle en C3-C12, et notamment le (méth)acrylate d'isobornyle, le (méth)acrylate d'isobutyle, le (méth)acrylate d'éthyl-2-hexyle, le (méth)acrylate de dodécyle, le (méth)acrylate de stéaryle, le (méth)acrylate de béhényle, le (méth)acrylate de méthyle, le (méth) acrylate de tertiobutyle;
- l'acide (méth)acrylique.

Et tout particulièrement parmi, seul ou en mélange, :

- les méthacrylates d'alkyle en C1-C4 ou de cycloalkyle en C3-C12, et notamment le méthacrylate de méthyle, le méthacrylate d'isobornyle, le méthacrylate d'isobutyle;
- les acrylates de tertiobutyle, l'acrylate de méthyle, l'acrylate d'isobutyle, l'acrylate d'isobornyle;
- l'acide acrylique et l'acide méthacrylique.

Dans un mode de réalisation préféré, le polymère séquencé comprend :

- une première séquence obtenue à partir d'au moins un monomère acrylate de formule $CH_2$=CH-$COOR_2$ dans laquelle $R_2$ représente un groupe cycloalkyle $C_4$ à $C_{12}$ et d'au moins un monomère méthacrylate de formule $CH_2$=C ($CH_3$)-COOR'$_2$

dans laquelle R'$_2$ représente un groupe cycloalkyle $C_4$ à $C_{12;}$ et d'au moins un monomère de formule (I); ladite séquence ayant une Tg strictement supérieure à 20°C, et

- une deuxième séquence obtenue à partir d'au moins un monomère acide (méth)acrylique et d'au moins un monomère additionnel dont l'homopolymère a une Tg inférieure ou égale à 20°C; de préférence ladite séquence ayant une Tg inférieure ou égale à 20°C.

Selon ce mode de réalisation, la première séquence peut être obtenue exclusivement à partir dudit monomère acrylate, dudit monomère méthacrylate et dudit monomère de formule (I).
Le monomère acrylate de formule $CH_2$=CH-$COOR_2$, seul ou en mélange, peut représenter 27 à 60% en poids, notamment 30 à 55% en poids, voire 33 à 50% en poids, du poids de ladite séquence.
Le monomère méthacrylate de formule $CH_2$=C($CH_3$)-COOR'$_2$, seul ou en mélange, peut représenter 27 à 60% en poids, notamment 30 à 55% en poids, voire 33 à 50% en poids, du poids de ladite séquence.
La proportion de la première séquence va avantageusement de 20 à 90% en poids du polymère, mieux de 30 à 80% et encore mieux de 60 à 80%.
La première séquence peut en outre comprendre de l'acide (méth)acrylique, de préférence de l'acide acrylique; de l'acrylate de tertiobutyle; un méthacrylate de formule $CH_2$ = C($CH_3$)-$COOR_1$ dans laquelle $R_1$ représente un groupe alkyle non substitué, linéaire ou ramifié, contenant de 1 à 4 atomes de carbone, tel qu'un groupe méthyle, éthyle, propyle ou isobutyle.
Selon ce mode de réalisation, la deuxième séquence est de préférence obtenue à partir d'acide (méth)acrylique et d'un monomère dont l'homopolymère a une Tg inférieure ou égale à 20°C, notamment l'acrylate d'isobutyle.
Chacune des première et/ou deuxième séquence, peut comprendre, outre les monomères indiqués ci-dessus, un ou plusieurs autres monomères appelés monomères additionnels, différents des monomères principaux cités précédemment. Les monomères additionnels peuvent représenter 0,5 à 30% en poids du poids du polymère. Préférentiellement, dans ce mode de réalisation, le polymère ne contient pas de monomère additionnel et est exclusivement constitué des monomères indiqués ci-dessus.
La masse molaire moyenne en poids (Mw) du polymère séquencé selon l'invention est de préférence comprise entre 25 000 et 1 000 000, mieux entre 30 000 et 750 000, voire entre 40 000 et 500 000, et préférentiellement entre 50 000 et 250000.
On détermine les masses molaires moyennes en poids (Mw) et en nombre (Mn) par chromatographie liquide par perméation de gel (solvant THF, courbe d'étalonnage établie avec des étalons de polystyrène linéaire, détecteur réfractométrique et UV).
De préférence, l'indice de polydispersité du polymère selon l'invention est supérieur à 2, par exemple allant de 2 à 9, de préférence supérieur ou égal à 2,5, par exemple allant de 2,5 à 8, et mieux allant de 2,8 à 7. L'indice de polydispersité Ip du polymère est égal au rapport de la masse moyenne en poids Mw sur la masse moyenne en nombre Mn.
Le polymère séquencé peut être obtenu par polymérisation radicalaire en solution selon le procédé de préparation suivant :

- on peut introduire une partie du solvant de polymérisation dans un réacteur adapté, on chauffe jusqu'à atteindre la température adéquate pour la polymérisation (typiquement entre 60 et 120°C),

- une fois cette température atteinte, on peut ajouter les monomères constitutifs de la première séquence, en présence d'une partie de l'initiateur de polymérisation,
- au bout d'un temps T correspondant à un taux de conversion maximum de 90% de préférence, on peut introduire les monomères constitutifs de la deuxième séquence et l'autre partie de l'initiateur,
- on laisse réagir le mélange pendant un temps T' (allant notamment de 3 à 6 heures) au bout duquel le mélange est ramené à température ambiante (25°C), de manière à obtenir le polymère en solution dans le solvant de polymérisation.

  Par solvant de polymérisation, on entend un solvant, ou un mélange de solvants, notamment choisi parmi l'acétate d'éthyle, l'acétate de butyle, les alcools en C1-C6 tels que l'isopropanol, l'éthanol, les alcanes aliphatiques tels que l'isododécane et leurs mélanges. De préférence, le solvant de polymérisation est un mélange d'acétate de butyle et d'isopropanol ou est l'isododécane.

De préférence, le polymère séquencé n'est pas hydrosoluble, c'est à dire qu'il n'est pas soluble dans l'eau ou dans un mélange d'eau et de monoalcools linéaires ou ramifiés ayant de 2 à 5 atomes de carbone comme l'éthanol, l'isopropanol ou le n-propanol, sans modification de pH, à une teneur en matière active d'au moins 1 % en poids, à température ambiante (25°C).

De préférence, ledit polymère séquencé, seul ou en mélange, peut être présent à raison de 1 à 45% en poids dans la composition selon l'invention, notamment 2 à 40% en poids, voire 3 à 35% en poids, par rapport au poids total de la composition.

Les compositions cosmétiques selon l'invention comprennent, outre lesdits polymères, un milieu physiologiquement acceptable, notamment cosmétiquement ou pharmaceutiquement acceptable, c'est-à-dire un milieu compatible avec les matières kératiniques telles que la peau du visage ou du corps, les lèvres, les cheveux, les cils, les sourcils et les ongles.

La composition selon l'invention peut avantageusement comprendre une phase grasse liquide, qui peut constituer un milieu solvant des polymères selon l'invention, et qui peut comprendre au moins un composé choisi parmi les huiles et/ou solvants d'origine minérale, animale, végétale ou synthétique, carbonés, hydrocarbonés, fluorés et/ou siliconés, volatils ou non volatils, seuls ou en mélange dans la mesure où ils forment un mélange homogène et stable et sont compatibles avec l'utilisation envisagée.

Par 'volatil', on entend au sens de l'invention, tout composé susceptible de s'évaporer au contact des matières kératiniques, ou des lèvres, en moins d'une heure, à température ambiante (25°C) et pression atmosphérique (1 atm). Notamment, ce composé volatil a une pression de vapeur non nulle, à température ambiante et pression atmosphérique, notamment allant de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg). Par opposition, on entend par 'non volatil', un composé restant sur les matières kératiniques ou les lèvres à température ambiante et pression atmosphérique, au moins une heure et ayant notamment une pression de vapeur inférieure à $10^{-3}$ mm de Hg (0,13Pa).

De préférence, le milieu physiologiquement acceptable de la composition selon l'invention peut comprendre, dans une phase grasse liquide, au moins une huile et/ou un solvant qui peut être choisi parmi, seul ou en mélange :

1/ les esters des acides monocarboxyliques avec les monoalcools et polyalcools; avantageusement, ledit ester est un benzoate d'alkyle en C12-C15 ou répond à la formule suivante : $R'_1$-COO-$R'_2$ où :

> $R'_1$ représente un radical alkyle linéaire ou ramifié de 1 à 40 atomes de carbone, de préférence de 7 à 19 atomes de carbone, comprenant éventuellement une ou plusieurs double liaisons éthyléniques, éventuellement substitué et dont la chaîne hydrocarbonée peut être interrompue par un ou plusieurs hétéroatomes choisis parmi N et O et/ou une ou plusieurs fonctions carbonyles, et
> $R'_2$ représente un radical alkyle linéaire ou ramifié de 1 à 40 atomes de carbone, de préférence de 3 à 30 atomes de carbone et mieux de 3 à 20 atomes de carbone, comprenant éventuellement une ou plusieurs double liaisons éthyléniques, éventuellement substitué et dont la chaîne hydrocarbonée peut être interrompue par un ou plusieurs hétéroatomes choisis parmi N et O et/ou une ou plusieurs fonctions carbonyles.

Par "éventuellement substitué", on entend que $R'_1$ et/ou $R'_2$ peuvent porter un ou plusieurs substituants choisis, par exemple, parmi les groupements comprenant un ou plusieurs hétéroatomes choisi parmi O et/ou N, tels que amino, amine, alcoxy, hydroxyle.

Des exemples des groupes $R'_1$ sont ceux dérivés des acides gras de préférence supérieur choisis dans le groupe constitué des acides acétique, propionique, butyrique, caproïque, caprylique, pélargonique, caprique, undécanoïque, laurique, myristique, palmitique, stéarique, isostéarique, arachidique, béhénique, oléique, linolénique, linoléïque, oléostéarique, arachidonique, érucique, et de leurs mélanges.

De préférence, $R'_1$ est un groupe alkyle ramifié non substitué de 4 à 14 atomes de carbone, de préférence de 8 à

10 atomes de carbone et $R_2$ est un groupe alkyle ramifié non substitué de 5 à 15 atomes de carbone, de préférence de 9 à 11 atomes de carbone.
On peut en particulier citer, de préférence, les esters en $C_8$-$C_{48}$, éventuellement incorporant dans leur chaîne hydrocarbonée un ou plusieurs hétéroatomes parmi N et O et/ou une ou plusieurs fonctions carbonyle; et plus particulièrement l'huile de purcellin (octanoate de cétostéaryle), l'isononanoate d'isononyle, le myristate d'isopropyle, le palmitate d'isopropyle, le palmitate d'éthyl-2-hexyle, le stéarate d'octyl-2-dodécyle, l'érucate d'octyl-2-dodécyle, l'isostéarate d'isostéaryle, le benzoate d'alcool en $C_{12}$ à $C_{15}$, le laurate d'hexyle, l'adipate de diisopropyle; et les heptanoates, octanoates, décanoates ou ricinoléates d'alcools ou de polyalcools, par exemple d'alcools gras comme le dioctanoate de propylène glycol, ainsi que le N-lauroyl sarcosinate d'isopropyle (notamment Eldew-205SL d'Ajinomoto); les esters hydroxylés comme le lactate d'isostéaryle, le malate de di-isostéaryle; et les esters du pentaérythritol; les esters ramifiés en C8-C16, notamment le néopentanoate d'isohexyle.

2/ les huiles végétales hydrocarbonées à forte teneur en triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées; ces huiles sont notamment les huiles de germe de blé, de maïs, de tournesol, de karité, de ricin, d'amandes douces, de macada-mia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat, de jojoba, de palme, de calophyllum; ou encore les triglycérides des acides caprylique/caprique comme ceux vendus par la société Stearinerie Dubois ou ceux vendus sous les dénominations "Miglyol 810®", "812® " et "818® " par la société Dynamit Nobel.

3/ les alcools, et notamment les monoalcools, en C6-C32, notamment C12-C26, comme l'alcool oléïque, l'alcool linoléique, l'alcool linolénique, l'alcool isostéarylique, le 2-hexyldécanol, le 2-butyloctanol, le 2-undécylpentadécanol et l'octyldodécanol;

4/ les huiles hydrocarbonées, linéaires ou ramifiés, volatiles ou non, d'origine synthétique ou minérale, qui peuvent être choisies parmi les huiles hydrocarbonées ayant de 5 à 100 atomes de carbones, et notamment la vaseline, les polydécènes, les polyisobutènes hydrogénés tel que le Parléam, le squalane, le perhydrosqualène et leurs mélanges. On peut plus particulièrement citer les alcanes linéaires, ramifiés et/ou cycliques en C5-C48, et préférentiellement les alcanes ramifiés en C8-C16 comme les isoalcanes en C8-C16 d'origine pétrolière ou non (appelées aussi isoparaffines); notamment le décane, l'heptane, l'undécane, le dodécane, le tridécane, le cyclohexane; ainsi que l'isododécane, l'isodécane, l'isohexadécane; et leurs mélanges.

5/ les huiles de silicone, volatiles ou non volatiles;
Comme huiles de silicone volatiles, on peut citer les huiles de silicones linéaires ou cycliques volatiles, notamment celles ayant une viscosité inférieure à 8 centistokes, et ayant notamment de 2 à 10 atomes de silicium, ces silicones comportant éventuellement des groupes alkyles ou alkoxy ayant de 1 à 22 atomes de carbone; et en particulier l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane, l'hexaméthyldisiloxane, l'octaméthyltrisiloxane, le décaméthyltétrasiloxane, le dodécaméthylpentasiloxane, le méthylhexyldiméthylsiloxane et leurs mélanges.
Les huiles de silicone non volatiles utilisables selon l'invention peuvent être les polydiméthylsiloxanes (PDMS), les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényltriméthicones, les phényldiméthicones, les phényltriméthylsiloxydiphénylsiloxanes, les diphényldiméthicones, les diphénylméthyldiphényltrisiloxanes, les 2phényléthyl triméthylsiloxysilicates.

Dans un mode de réalisation préféré, les huiles volatiles, notamment carbonées, seules ou en mélange, sont présentes dans la composition en une quantité comprise entre 30 et 80% en poids, notamment 35 à 75% en poids, voire 40 à 70% en poids, par rapport au poids total de la composition.
La phase grasse liquide peut en outre comprendre des huiles et/ou solvants additionnels, qui peuvent être choisis parmi, seul ou en mélange :

- les huiles fluorées telles que les perfluoropolyéthers, les perfluoroalcanes comme la perfluorodécaline, les perfluorodamantanes, les monoesters, diesters et triesters de perfluoroalkylphosphates et les huiles esters fluorés;
- les huiles d'origine animale;
- les éthers en $C_6$ à $C_{40}$, notamment en C10-C40; les éthers de propylène glycol liquides à température ambiante tels que le monométhyléther de propylène glycol, l'acétate de monométhyléther de propylène glycol, le mono n-butyl éther de dipropylène glycol ;

- les acides gras en $C_8$-$C_{32}$, comme l'acide oléique, l'acide linoléique, l'acide linolénique et leurs mélanges.
- les huiles bifonctionnelles, comprenant deux fonctions choisies parmi ester et/ou amide et comprenant de 6 à 30 atomes de carbone, notamment 8 à 28 atomes de carbone, mieux de 10 à 24 carbones, et 4 hétéroatomes choisis parmi O et N; de préférence les fonctions amide et ester étant dans la chaîne;
- les cétones liquides à température ambiante (25°C) tels que la méthyléthylcétone, la méthylisobutylcétone, la diisobutylcétone, l'isophorone, la cyclohexanone, l'acétone ;
- les aldéhydes liquides à température ambiante tels que le benzaldéhyde, l'acétaldéhyde.

La phase grasse liquide peut représenter 5 à 90% en poids de la composition, notamment de 10 à 75% en poids, en particulier de 15 à 60% en poids, voire de 25 à 55% en poids, du poids total de la composition.

La composition selon l'invention peut également comprendre un ou plusieurs solvants organiques, physiologiquement acceptables.

Ces solvants peuvent être généralement présents en une teneur allant de 0,1 à 90%, de préférence de 0,5 à 85%, de préférence encore de 10 à 80% en poids, par rapport au poids total de la composition, et mieux de 30 à 50 %.

On peut notamment citer, outre les solvants organiques hydrophiles cités plus haut, les cétones liquides à température ambiante tels que méthyléthylcétone, méthylisobutylcétone, diisobutylcétone, l'isophorone, la cyclohexanone, l'acétone; les éthers de propylène glycol liquides à température ambiante tels que le monométhyléther de propylène glycol, l'acétate de monométhyléther de propylène glycol, le mono n-butyl éther de dipropylène glycol; les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle; les éthers liquides à 25°C tels que le diéthyléther, le diméthyléther ou le dichlorodiéthyléther; les alcanes liquides à 25°C tels que le décane, l'heptane, le dodécane, l'isododécane, le cyclohexane; les composés cycliques aromatiques liquides à 25°C tels que le toluène et le xylène; les aldéhydes liquides à 25°C tels que le benzaldéhyde, l'acétaldéhyde et leurs mélanges.

La composition peut également comprendre des corps gras solides à température ambiante tels que les cires, les corps gras pâteux, les gommes et leurs mélanges. Ils peuvent être d'origine animale, végétale, minérale ou synthétique.

Par cire au sens de la présente invention, on entend un composé lipophile, solide à température ambiante (25°C), à changement d'état solide/liquide réversible, ayant un point de fusion supérieur ou égal à 25°C pouvant aller jusqu'à 120°C. En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METLER. Les cires peuvent être hydrocarbonées, fluorées et/ou siliconées et être d'origine végétale, minérale, animale et/ou synthétique. En particulier, les cires présentent un point de fusion supérieur à 30°C et mieux supérieur à 45°C. Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxydiméthicone ayant de 16 à 45 atomes de carbone.

Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.

Par corps gras pâteux, on entend un produit visqueux contenant une fraction liquide et une fraction solide. On entend notamment des corps gras ayant un point de fusion allant de 20 à 55°C et/ou une viscosité à 40°C allant de 0,1 à 40 Pa.s (1 à 400 poises) mesurée au Contraves TV ou Rhéomat 80. L'homme du métier peut choisir le mobile permettant de mesurer la viscosité, parmi les mobiles MS-r3 et MS-r4, sur la base de ses connaissances générales, de manière à pouvoir réaliser la mesure de la viscosité du composé pâteux testé. Les valeurs de point de fusion correspondent, selon l'invention, au pic de fusion mesurée par la méthode "Differential Scanning Calorimetry" avec une montée en température de 5 ou 10 °C/min. De préférence, ces corps gras sont des composés hydrocarbonés (contenant principalement des atomes de carbone et d'hydrogène et éventuellement des groupements ester), éventuellement de type polymérique; ils peuvent également être choisis parmi les composés siliconés et/ou fluorés; ils peuvent aussi se présenter sous forme d'un mélange de composés hydrocarbonés et/ou siliconés et/ou fluorés. Dans le cas d'un mélange de différents corps gras pâteux, on utilise de préférence les composés pâteux hydrocarbonés en proportion majoritaire. Parmi les composés pâteux susceptibles d'être utilisés dans la composition selon l'invention, on peut citer les lanolines et les dérivés de lanoline comme les lanolines acétylées ou les lanolines oxypropylènées ou le lanolate d'isopropyle; des esters d'acides ou d'alcools gras, notamment ceux ayant 20 à 65 atomes de carbone comme le citrate de triisostéaryle ou de cétyle; le propionate d'arachidyle; le polylaurate de vinyle; les esters du cholestérol comme les triglycérides d'origine végétale tels que les huiles végétales hydrogénées, les polyesters visqueux comme l'acide poly(12-hydroxystéarique) et leurs mélanges. Comme triglycérides d'origine végétale, on peut utiliser les dérivés d'huile de ricin hydrogénée. On peut aussi citer les corps gras pâteux siliconés tels que les polydiméthylsiloxanes (PDMS) ayant des chaînes pendantes du type alkyle ou alcoxy ayant de 8 à 24 atomes de carbone, comme les stéaryl diméthicones.

La nature et la quantité des corps solides sont fonction des propriétés mécaniques et des textures recherchées. A titre indicatif, la composition peut contenir de 0,1 à 50% en poids de cires, par rapport au poids total de la composition et mieux de 1 à 30% en poids.

La composition peut aussi comprendre un milieu hydrophile comprenant de l'eau ou un mélange d'eau et d'un ou plusieurs solvants organiques hydrophiles comme les alcools et notamment les monoalcools inférieurs linéaires ou ramifiés ayant de 2 à 5 atomes de carbone comme l'éthanol, l'isopropanol ou le n-propanol; les polyols comme la glycérine, la diglycérine, le propylène glycol, le sorbitol, le pentylène glycol; les polyéthylène glycols, ou bien encore des éthers en $C_2$ et des aldéhydes en $C_2$-$C_4$ hydrophiles. L'eau ou le mélange d'eau et de solvants organiques hydrophiles peut être présent dans la composition selon l'invention en une teneur de 10 à 80% en poids, par rapport au poids total de la composition. La composition peut également être anhydre.

La composition selon l'invention peut également comprendre une ou plusieurs matières colorantes choisies parmi les composés pulvérulents comme les pigments, les charges, les nacres et les paillettes, et/ou les colorants liposolubles ou hydrosolubles.

Les matières colorantes, notamment pulvérulentes, peuvent être présentes, dans la composition, en une teneur de 0,01 à 50% en poids, par rapport au poids de la composition, de préférence de 0,1 à 40% en poids, voire de 1 à 30% en poids.

Par pigments, il faut comprendre des particules de toute forme, blanches ou colorées, minérales ou organiques, insolubles dans le milieu physiologique, destinées à colorer la composition.

Par nacres, il faut comprendre des particules de toute forme irisées, notamment produites par certains mollusques dans leur coquille ou bien synthétisées.

Les pigments peuvent être blancs ou colorés, minéraux et/ou organiques, interférentiels ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être minérales ou organiques, lamellaires ou sphériques. On peut citer le talc, le mica, la silice, le kaolin, les poudres de Nylon et de polyéthylène, de poly-β-alanine et de polyéthylène, le Téflon, la lauroyl-lysine, l'amidon, le nitrure de bore, les poudres de polymères de tétrafluoroéthylène, les microsphères creuses telles que l'Expancel (Nobel Industrie), le polytrap (Dow Corning) et les microbilles de résine de silicone (Tospearls de Toshiba, par exemple), le carbonate de calcium précipité, le carbonate et l'hydrocarbonate de magnésium, l'hydroxyapatite, les microsphères de silice creuses (SILICA BEADS de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les colorants liposolubles sont par exemple le rouge Soudan, le DC Red 17, le DC Green 6, le β-carotène, l'huile de soja, le brun Soudan, le DC Yellow, 11, le DC Violet 2, le DC orange 5, le jaune quinoléine. Ils peuvent représenter 0,01 à 20% du poids de la composition et mieux de 0,1 à 6 %.

Les colorants hydrosolubles sont par exemple le jus de betterave, le bleu de méthylène et peuvent représenter 0,01 à 6% du poids total de la composition.

La composition selon l'invention peut comprendre en outre en outre une ou plusieurs charges, notamment en une teneur allant de 0,01 % à 50% en poids, par rapport au poids total de la composition, de préférence allant de 0,02% à 30% en poids. Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage. Les charges peuvent être minérales ou organiques de toute forme, plaquettaires, sphériques ou oblongues. On peut citer le talc, le mica, la silice, le kaolin, les poudres de polyamide (Nylon®), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition peut comprendre en outre un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques. Parmi les polymères filmogènes susceptibles d'être utilisés dans la composition de la présente invention, on peut citer les polymères synthétiques, de type radicalaire ou de type polycondensat, les polymères d'origine naturelle et leurs mélanges, en particulier les polymères acryliques, les polyuréthanes, les polyesters, les polyamides, les polyurées, les polymères cellulosiques comme la nitrocellulose.

La composition selon l'invention peut également comprendre des ingrédients couramment utilisés en cosmétique, tels que les vitamines, les épaississants, les gélifiants, les oligo-éléments, les adoucissants, les séquestrants, les parfums, les agents alcalinisants ou acidifiants, les conservateurs, les filtres solaires, les tensioactifs, les antioxydants, les agents anti-chutes des cheveux, les agents antipelliculaires, les agents propulseurs, les céramides, les agents auxiliaires de filmification, ou leurs mélanges.

Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention peut se présenter sous la forme d'une suspension, une dispersion notamment d'huile dans de l'eau grâce à des vésicules; une solution aqueuse ou huileuse éventuellement épaissie voire gélifiée; une émulsion huile-dans-eau, eau-dans-huile, ou multiple; un gel ou une mousse; un gel huileux ou émulsionné; une dispersion de vésicules notamment lipidiques; une lotion biphase ou multiphase; un spray; une poudre libre, compacte ou coulée; une pâte anhydre. Cette composition peut avoir l'aspect d'une lotion, d'une crème, d'une pommade, d'une pâte souple, d'un onguent, d'une mousse, d'un solide coulé ou moulé et notamment en stick ou en coupelle, ou encore de solide compacté.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition cosmétique selon l'invention peut se présenter sous la forme d'un produit de soin et/ou de maquillage de la peau du corps ou du visage, des lèvres, des ongles, des cils, des sourcils et/ou des cheveux, d'un produit solaire ou autobronzant, d'un produit capillaire pour le soin, le traitement, la mise en forme, le maquillage ou la coloration des cheveux.

Elle peut ainsi se présenter sous la forme d'une composition de maquillage, notamment un produit pour le teint tel qu'un fond de teint, un fard à joues ou à paupières; un produit pour les lèvres tel qu'un rouge à lèvres, un gloss ou un soin des lèvres; un produit anti-cernes; un blush, un mascara, un eye-liner; un produit de maquillage des sourcils, un crayon à lèvres ou à yeux; un produit pour les ongles tel qu'un vernis à ongles ou un soin des ongles; un produit de maquillage du corps; un produit de maquillage des cheveux (mascara ou laque pour cheveux).

Elle peut également se présenter sous forme d'une composition de protection ou de soin de la peau du visage, du cou, des mains ou du corps, notamment une composition antirides, une composition hydratante ou traitante; une composition solaire ou de bronzage artificiel (autobronzant).

Elle peut encore se présenter sous forme d'un produit capillaire, notamment pour la coloration, le maintien de la coiffure, la mise en forme des cheveux, le soin, le traitement ou le nettoyage des cheveux, telle que des shampooings, des aprèsshampoings, des gels, des lotions de mise en plis, des lotions pour le brushing, des compositions de fixation et de coiffage telles que les laques ou spray.

De préférence, la composition cosmétique selon l'invention se présente sous la forme d'un produit de maquillage, notamment d'un rouge à lèvres, d'un gloss (brillant à lèvres), d'un mascara, d'un vernis à ongles, d'un fond de teint, ou d'un produit de soin tel qu'une crème de soin pour le visage ou un produit solaire.

L'invention a encore pour objet un procédé de traitement cosmétique, notamment de maquillage ou de soin, des matières kératiniques telles que la peau du corps ou du visage, des lèvres, des ongles, des cheveux, des sourcils et/ou des cils, comprenant l'application sur lesdites matières d'une composition cosmétique telle que définie précédemment.

Ce procédé permet notamment le maquillage de la peau, des cils, des ongles, des cheveux et/ou des lèvres.

L'invention est illustrée plus en détail dans les exemples suivants.

<u>Brillance mesurée au brillancemètre sur un dépôt sec de polymère</u>

La brillance peut être mesurée à l'aide d'un brillancemètre, de manière conventionnelle par la méthode suivante. Sur une carte de contraste de marque LENETA et de référence FORM 1A PENOPAC, on étale une couche de 50 μm d'épaisseur de polymère à tester, en solution à 50% dans l'isododécane, à l'aide d'un étaleur. La couche recouvre au moins le fond noir de la carte. On laisse sécher le dépôt 24 heures à une température de 25°C, puis on procède à la mesure de la brillance à 20° sur le fond noir à l'aide d'un brillancemètre de marque DR LANGE, REF03. On procède

également à la mesure de la brillance à 60° comme précédemment.

Tenue à l'huile d'olive par mesure de l'aspect collant

Elle est déterminée avec une goutte d'huile d'olive mise sur un film de polymère sec.
Un film de polymère est réalisé à partir d'une solution à 20% en poids de polymère séquencé dans l'isododécane; 0,5 ml est étalé sur une plaque de verre de 2,5 x 7,5 cm et laissé sécher à température ambiante (25°C) pendant 24 heures. Ensuite, 1 ml d'huile d'olive est étalé sur le film.
Après le temps voulu (2 heures), l'excès d'huile est essuyé du film et l'aspect collant est évalué au toucher.
Une note + est donnée lorsque le collant est perceptible après une pression courte (environ 1 seconde) avec le doigt.
Une note - est donnée lorsque aucun collant n'est détecté, après une pression courte avec le doigt.
Le collant traduit la sensibilité du polymère à l'huile d'olive. Plus il est important, plus le polymère est sensible à l'huile et donc plus le dépôt sera facilement altéré, par exemple lors des repas (en présence d'huile alimentaire) ou par le sébum. Il en résulte une moins bonne tenue du polymère sur la peau. Il en résulte aussi une diminution du confort: plus le film est collant, plus la composition est inconfortable à porter.

Détermination du caractère cassant

Sur une carte de contraste de marque LENETA et de référence FORM 1A PENOPAC, on étale une couche de 50 μm d'épaisseur de polymère en solution à 50% dans l'isododécane, à l'aide d'un étaleur. La couche recouvre au moins le fond noir de la carte. On laisse sécher le dépôt 24 heures à une température de 25°C. On courbe la carte et on observe la formation de fissures.
Une note +++ signifie que le film est très cassant, les morceaux se détachent de la carte, le film craque sans même que la carte ne soit courbée.
Une note + signifie que le film est peu cassant, quelques fissures apparaissent, le film reste cohésif.

Méthode de mesure de la viscosité des polymères

La viscosité à 25°C du polymère séquencé est mesurée à l'aide d'un viscosimètre cylindrique de type BROOKFIELD DV-I+.

**Procédé de synthèse général des polymères**

1/ synthèse du comparatif 1 poly(acrylate d'isobornyle/méthacrylate d'isobornyle /acrylate isobutyle/acide acrylique)

On introduit 300 g d'isododécane dans un réacteur de 1 litre, puis on augmente la température de façon à passer de la température ambiante (25°C) à 90°C en 1 heure. On ajoute, à 90°C et en 1 heure, 105 g de méthacrylate d'isobornyle, 105 g d'acrylate d'isobornyle et 1,8 g de 2,5-bis(2-éthylhexanoylperoxy)-2,5-diméthylhexane (Trigonox®141 d'Akzo Nobel). On maintient le mélange pendant 1 h30 à 90°C.
On introduit ensuite au mélange précédent, toujours à 90°C et en 30 minutes, 75 g d'acrylate d'isobutyle, 15 g d'acide acrylique et 1,2 g de 2,5-bis(2éthylhexanoylperoxy)-2,5-diméthylhexane. Le mélange est maintenu 3 heures à 90°C, puis l'ensemble est refroidi.
On obtient une solution à 50% de matière sèche de polymère dans l'isododécane. Le polymère comprend une première séquence poly(acrylate d'isobornyle/méthacrylate d'isobornyle) ayant une Tg de 128°C, une deuxième séquence poly (acrylate d'isobutyle/acide acrylique) ayant une Tg de -9°C et une séquence intermédiaire qui est un polymère statistique acrylate d'isobornyle/méthacrylate d'isobornyle /acrylate d'isobutyle/acide acrylique. Il s'agit de Tg théoriques calculées par la loi de FOX.
La solution à 50% en poids de polymère dans l'isododécane a une viscosité de 30 000 cps à 25°C.

2/ synthèse des polymères selon l'invention

Les quantités de monomères sont adaptées pour les synthèses des polymères selon l'invention, qui sont réalisées selon le mode opératoire précédent.

**Exemple 1**

Selon le mode opératoire général décrit ci-dessus, on prépare les polymères suivants (% en poids):

| | Séquence de Tg > 20°C | | | Séquence de Tg ≤ 20°C | |
|---|---|---|---|---|---|
| | Acrylate d'isobornyle | Méthacrylate d'isobornyle | MPEG 350* | Acrylate d'isobutyle | Acide acrylique |
| Comparatif 1 | 35 | 35 | - | 25 | 5 |
| Comparatif 2 | 30 | 30 | 10 | 25 | 5 |
| Exemple 1 (invention) | 32,5 | 32,5 | 5 | 25 | 5 |
| *MPEG 350 : méthacrylate de méthoxypolyethylène glycol de masse molaire 350 g/mol (Bisomer MPEG 350 de Cognis) | | | | | |

**Exemple 2**

On détermine la brillance, le caractère collant ainsi que le cassant, des polymères ci-dessus, en comparaison avec le polymère comparatif.

| | Comparatif 1 | Comparatif 2 | Exemple 1 |
|---|---|---|---|
| viscosité (25°C) de la solution | 30 000 cps | Polymère solide à froid; non véhiculable dans l'isododécane; donc pas de mesures possibles | 18 300 cps |
| Aspect du film | Brillant | | brillant |
| Brillance | 20°=79,6 60°=88,7 | | 20°=77,9 60°=86,9 |
| Collant (sensibilité à l'huile d'olive) | 2 heures : + | | 2 heures : - |
| Cassant | +++ | | + |

On constate donc que les films selon l'invention sont moins collants et moins cassants que les films obtenus avec le polymère comparatif.
Par ailleurs, la présence de MPEG permet d'obtenir des polymères dont la viscosité est plus basse, en comparaison des polymères de l'art antérieur; ceci peut faciliter la mise en oeuvre de ces polymères, notamment la facilité de manipulation, d'incorporation dans une composition.

**Exemple 3**

A partir d'une solution à 50% dans l'isododécane, on réalise sur carte Leneta un film d'épaisseur 50 μm (épaisseur 'humide' c'est-à-dire de la solution telle que déposée). On étale une goutte d'eau sur chaque film et on laisse en contact pendant 24 heures. Après essuyage de la goutte d'eau, on évalue au toucher la surface en contact.
Pour les films obtenus avec la solution de polymère selon l'invention, la surface est glissante et souple (non cassante).
Pour les films obtenus avec la solution de polymère comparatif, la surface est cassante et non glissante.

**Exemple 4 : Composition de mascara**

On prépare un mascara ayant la composition suivante :

| | |
|---|---|
| Cires (abeille, paraffine, carnauba) | 17 g |
| Hectorite modifiée (Bentone® 38V) | 5,3 g |
| Carbonate de propylène | 1,7 g |
| Pigments | 5 g |
| Solution de polymère de l'exemple 1 | 20 g (soit 10 g MS*) |
| Isododécane | qsp 100 g |
| *MS : matière sèche | |

Le mascara, après application sur les cils, est jugé très satisfaisant.

**Exemple 5 : Stick de rouge à lèvres**

On prépare la composition de rouge à lèvres suivante (% en poids):

| | |
|---|---|
| Cire de polyéthylène | 15 % |
| Solution de polymère de l'exemple 1 | 20 % (soit 10% MS) |
| Polyisobutène hydrogéné (Parléam de Nippon Oil Fats) | 26 % |
| Pigments | 8.6 % |
| Isododécane | qsp 100% |

La composition obtenue après application sur les lèvres présente de bonnes propriétés cosmétiques.

**Exemple 6 : Fond de teint**

On prépare un fond de teint comprenant les composés suivants :

| | | |
|---|---|---|
| Phase A | Cetyl Dimethicone copolyol | 3 g |
| | (ABIL EM 90 de la société GOLDSCHMIDT) Succinate d'isostéaryl diglycéryle | 0,6 g |
| | (IMWITOR 780K de la société CONDEA) Isododécane | 18,5 g |
| | Pigments | 10 g |
| | Solution de polymère de l'exemple 1 | 16 g (soit 8 g MS) |
| | Poudre de polyamide (NYLON-12) | 8 g |
| Phase B | Eau | qsp 100 g |
| | Sulfate de magnésium | 0,7 g |
| | Conservateur | qs |
| Phase C | Eau | 2 g |
| | Conservateur | qs |

La composition obtenue présente de bonnes propriétés cosmétiques.

**Exemple 7 : Poudre compacte**

On prépare une poudre compactée ayant la composition suivante :

| | |
|---|---|
| *Composition A :* | |
| - Talc | 30 g |
| - Oxychlorure de bismuth | 10 g |
| - Stéarate de zinc | 4 g |
| - Poudre de Nylon | 20 g |
| - Solution du polymère de l'exemple 1 | 5 g (soit 2,5 g MS) |
| *Composition B :* | |
| - Oxydes de fer | 2 g |
| - Huile de vaseline | 6 g |

La poudre est obtenue de la façon suivante : on broie la composition A dans un broyeur de type KENWOOD pendant environ 5 minutes sous faible agitation, on ajoute la composition B et on broie l'ensemble environ 2 minutes à la même vitesse, puis 3 minutes à une vitesse plus rapide. On tamise ensuite la préparation sur un tamis de 0,16 mm, puis on compacte ce mélange dans des coupelles.
On obtient une poudre compactée présentant de bonnes propriétés cosmétiques.

**Exemple 8 : Gel pour le visage**

On prépare la composition suivante :

- vaseline (cire) 5 g
- hectorite modifiée (argile) 0,15 g
- ozokérite (cire) 5 g
- septaoléate de sorbitane oxyéthyléné (400E) 5 g
- solution du polymère de l'exemple 1 50 g (soit 25 g MS)
- palmitate d'isopropyle qsp 100 g

On obtient un gel ayant de bonnes propriétés cosmétiques.

**Exemple 9 : huile de soin**

On prépare la composition suivante :

- solution du polymère de l'exemple 1 50 g (soit 25 g MS)
- huile de soja 15 g
- huile de jojoba qsp 100 g

On obtient une huile de soin qui peut être appliquée sur le corps ou le visage.

**Exemple 10 : Gloss pour les lèvres**

On prépare un gloss ayant la composition suivante :

- polybutène 34%
- isononanoate d'isononyle 4%
- octyldodécanol 10%
- silice (Aérosil R972) 5%
- solution du polymère de l'exemple 1 28% (soit 14% MS)
- trimellitate de tridécyle qsp 100%

**Revendications**

1. Polymère séquencé comprenant au moins une première séquence et au moins une deuxième séquence, incompatibles l'une avec l'autre, dans lequel l'une des séquences présente une température de transition vitreuse (Tg) strictement supérieure à 20°C et comprend 0,5 à 13% en poids, par rapport au poids de ladite séquence, d'au moins un monomère de formule (I):

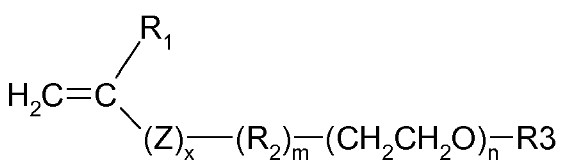

(I)

dans laquelle :

- R1 est un atome d'hydrogène ou un radical méthyle;
- Z est un groupement divalent choisi parmi -COO-, -CONH-, $-CONCH_3-$, -OCO-, - O-, $-SO_2-$ -CO-O-CO- ou $-CO-CH_2-CO-$;
- x est 0 ou 1;
- R2 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;
- m est 0 ou 1;
- n est un entier compris entre 3 et 300 inclus;

- R3 est un atome d'hydrogène ou un radical carboné, saturé ou insaturé, éventuellement aromatique, linéaire, ramifié ou cyclique, de 1 à 30 atomes de carbone, pouvant comprendre 1 à 20 hétéroatomes choisis parmi O, N, S, F, Si et P.

**2.** Polymère selon la revendication 1, dans lequel lesdites première et deuxième séquences sont reliées entre elles par un segment intermédiaire comprenant au moins un monomère constitutif de la première séquence et au moins un monomère constitutif de la deuxième séquence.

**3.** Polymère selon l'une des revendications précédentes, comprenant au moins une séquence ayant une Tg inférieure ou égale à 20°C.

**4.** Polymère selon l'une des revendications précédentes, dans lequel dans la formule (I) :

- x = 1 et Z représente COO ou CONH; et/ou
- R2 représente un radical alkylène ayant 1 à 20 atomes de carbone; un radical phénylène $-C_6H_4-$(ortho, méta ou para) éventuellement substitué, par un radical alkyle en C1-C12 comprenant éventuellement 1 à 18 hétéroatomes choisis parmi O, N, S, F, Si et P; ou un radical benzylène $-C_6H_4-CH_2-$ éventuellement substitué par un radical alkyle en C1-C12 comprenant éventuellement 1 à 18 hétéroatomes choisis parmi O, N, S, F, Si et P; et/ou
- n est compris entre 5 et 200 inclus, et/ou
- R3 est un atome d'hydrogène; un radical phényle éventuellement substitué par un radical alkyle en C1-C12 comprenant éventuellement 1 à 20 hétéroatomes choisis parmi O, N, S, F, Si et P; un radical alkyle en C1-C30, notamment C1-C22, voire C2-C16, comprenant éventuellement 1 à 18 hétéroatomes choisis parmi O, N, S, F, Si et P; un radical cycloalkyle en C3-C12, notamment C4-C8, voire C5-C6, comprenant éventuellement 1 à 18 hétéroatomes choisis parmi O, N, S, F, Si et P.

**5.** Polymère selon l'une des revendications précédentes, dans lequel les monomères de formule (I) sont tels que :

- x=1 et Z représente COO, et
- m=0, et
- n= 6 à 120 inclus, et
- R3 est choisi parmi un atome d'hydrogène; un radical phényle éventuellement substitué par un radical alkyle en C1-C12; un radical alkyle en C1-C30, notamment C1-C22, voire C2-C16.

**6.** Polymère selon l'une des revendications précédentes, dans lequel le monomère de formule (I) est choisi parmi :

- le (méth)acrylate de poly(éthylène glycol) dans lequel R1 est H ou méthyle; Z est COO, x = 1, m=0 et R3 = H;
- le (méth)acrylate de méthyl-poly(éthylène glycol), aussi appelé (méth)acrylate de méthoxy-poly(éthylèneglycol), dans lequel R1 est H ou méthyle, Z est COO, x = 1, m=0 et R3 = méthyle;
- les (méth)acrylate d'alkyl-poly(éthylène glycol) dans lequel R1 est H ou méthyle, Z est COO, x = 1, m=0 et R3 = alkyl.
- les (méth)acrylates de phényl-poly(éthylène glycol), aussi appelé (méth)acrylate de poly(éthylène glycol) phényl éther, dans lequel R1 est H ou méthyl, Z est COO, x = 1, m=0 et R3 = phényle.

**7.** Polymère selon l'une des revendications précédentes, dans lequel le monomère de formule (I) ou le mélange de tels monomères, représente 1 à 12% en poids, notamment 2 à 11 % en poids, voire 3 à 10% en poids, du poids total de monomères servant à former la séquence de Tg supérieure à 20°C.

**8.** Polymère selon l'une des revendications précédentes, présentant une masse molaire moyenne en poids (Mw) comprise entre 25 000 et 1 000 000, mieux entre 30 000 et 750 000, voire entre 40 000 et 500 000, et préférentiellement entre 50 000 et 250000.

**9.** Polymère selon l'une des revendications précédentes, présentant un indice de polydispersité supérieur à 2, par exemple allant de 2 à 9, de préférence supérieur ou égal à 2,5, par exemple allant de 2,5 à 8, et mieux allant de 2,8 à 7.

**10.** Composition cosmétique comprenant, dans un milieu cosmétiquement acceptable, au moins un polymère séquencé tel que défini à l'une des revendications 1 à 9.

**11.** Composition selon la revendication 10, dans laquelle le polymère séquencé, seul ou en mélange, est présent à raison de 1 à 45% en poids dans la composition selon l'invention, notamment 2 à 40% en poids, voire 3 à 35% en poids, par rapport au poids total de la composition.

**12.** Composition selon l'une des revendications 10 à 11, dans laquelle le milieu cosmétiquement acceptable comprend au moins un ingrédient choisi parmi : les huiles et/ou solvants d'origine minérale, animale, végétale ou synthétique, carbonés, hydrocarbonés, fluorés et/ou siliconés, volatils ou non volatils; les corps gras solides à température ambiante tels que les cires, les corps gras pâteux, les gommes; de l'eau; des solvants organiques hydrophiles; les matières colorantes; les polymères; les vitamines, les épaississants, les gélifiants, les oligo-éléments, les adoucissants, les séquestrants, les parfums, les agents alcalinisants ou acidifiants, les conservateurs, les filtres solaires, les tensioactifs, les antioxydants, les agents anti-chutes des cheveux, les agents antipelliculaires, les agents propulseurs, les céramides, les agents auxiliaires de filmification, ou leurs mélanges.

**13.** Composition selon l'une des revendications 10 à 12, se présentant sous la forme d'un produit de soin et/ou de maquillage de la peau du corps ou du visage, des lèvres, des ongles, des cils, des sourcils et/ou des cheveux, d'un produit solaire ou autobronzant, d'un produit capillaire pour le soin, le traitement, la mise en forme, le maquillage ou la coloration des cheveux.

**14.** Composition selon l'une des revendications 10 à 13, se présentant sous la forme d'un produit de maquillage, notamment d'un rouge à lèvres, d'un gloss (brillant à lèvres), d'un mascara, d'un vernis à ongles, d'un fond de teint, ou d'un produit de soin tel qu'une crème de soin pour le visage ou un produit solaire.

**15.** Procédé de traitement cosmétique des matières kératiniques comprenant l'application sur lesdites matières d'une composition cosmétique telle que définie à l'une des revendications 10 à 14.

Europäisches Patentamt
European Patent Office
Office européen des brevets

# RAPPORT DE RECHERCHE EUROPEENNE

Numéro de la demande
EP 09 16 3112

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | EP 1 582 540 A (OREAL [FR]) 5 octobre 2005 (2005-10-05) * page 8, ligne 42 - ligne 50 * * page 9, ligne 16 - ligne 17 * * alinéas [0003], [0046] - [0050] * ----- | 1-15 | INV. C08F290/06 C08F293/00 C08L53/00 A61K8/90 A61Q1/00 A61Q3/02 A61Q5/00 |
| A | US 2005/220729 A1 (LUUKAS TIMO [FR] ET AL) 6 octobre 2005 (2005-10-06) * alinéas [0027], [0065] - [0068], [0076], [0174] * ----- | 1-15 | |
| A | WO 03/046032 A (OREAL [FR]; MOUGIN NATHALIE [FR]; LION BERTRAND [FR]) 5 juin 2003 (2003-06-05) * page 3, ligne 30 - page 4, ligne 4 * * page 4, ligne 26 - page 5, ligne 3 * * page 7, ligne 11 - ligne 25 * * page 16, ligne 3 - ligne 7 * * page 16, ligne 15 - ligne 26 * * page 17, ligne 9 - ligne 12; revendications 15,17,22,33,38,50 * ----- | 1-15 | |
| | | | DOMAINES TECHNIQUES RECHERCHES (IPC) |
| A | WO 97/35541 A (OREAL [FR]; SEBAG HENRI [FR]; MOUGIN NATHALIE [FR]; LION BERTRAND [FR]) 2 octobre 1997 (1997-10-02) * page 2, ligne 23 - page 3, ligne 2 * * page 5, ligne 31 - page 6, ligne 9 * * page 8, ligne 4 - ligne 5; revendication 22 * ----- | 1-15 | C08F |

Le présent rapport a été établi pour toutes les revendications

1

| Lieu de la recherche | Date d'achèvement de la recherche | Examinateur |
|---|---|---|
| Munich | 2 juillet 2009 | Iraegui Retolaza, E |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 09 16 3112

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

02-07-2009

| Document brevet cité au rapport de recherche | Date de publication | Membre(s) de la famille de brevet(s) | Date de publication |
|---|---|---|---|
| EP 1582540 A | 05-10-2005 | FR 2868076 A1 | 30-09-2005 |
| | | JP 2005281697 A | 13-10-2005 |
| US 2005220729 A1 | 06-10-2005 | AUCUN | |
| WO 03046032 A | 05-06-2003 | AU 2002365296 A1 | 10-06-2003 |
| | | EP 1448646 A2 | 25-08-2004 |
| | | JP 2006514701 T | 11-05-2006 |
| WO 9735541 A | 02-10-1997 | AT 217781 T | 15-06-2002 |
| | | CA 2250097 A1 | 02-10-1997 |
| | | DE 69712751 D1 | 27-06-2002 |
| | | DE 69712751 T2 | 21-11-2002 |
| | | EP 0895467 A1 | 10-02-1999 |
| | | ES 2180025 T3 | 01-02-2003 |
| | | FR 2746640 A1 | 03-10-1997 |
| | | JP 3648530 B2 | 18-05-2005 |
| | | JP 11507065 T | 22-06-1999 |
| | | US 6403106 B1 | 11-06-2002 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1411069 A


### Littérature non-brevet citée dans la description


- Polymer Handbook. John Wiley, 1999